# EUROPÄISCHE PATENTSCHRIFT

(11) **EP 1 133 252 B1**
(45) Veröffentlichungstag und Bekanntmachung des Hinweises auf die Patenterteilung: **17.12.2003**
(21) Anmeldenummer: 98962399.6
(22) Anmeldetag: 27.11.1998
(51) Int. Cl.: A61B 5/00

(54) **BIOFEEDBACK-VERFAHREN**
BIOFEEDBACK METHOD
PROCEDE DE BIOFEEDBACK

(43) Veröffentlichungstag der Anmeldung: 19.09.2001
(73) Patentinhaber: Stephan, Egon Prof.Dr., 51427 Bergisch-Gladbach (DE)
(72) Erfinder: Stephan, Egon Prof.Dr., 51427 Bergisch-Gladbach (DE)
(74) Vertreter: von Kirschbaum, Albrecht, Dipl.-Ing.
(86) Internationale Anmeldenummer: EP9807685
(87) Internationale Veröffentlichungsnummer: WO00032089

(56) Entgegenhaltungen:
- EP-A- 0 872 255
- DE-A- 19 745 508
- US-A- 5 213 338
- US-A- 5 720 619
- DATABASE WPI Section PQ, Week 8336, 19. Oktober 1983 (1983-10-19) Derwent Publications Ltd., London, GB; Class P36, AN 83-757422 XP002110492 & SU 971 372 A (ROST MED INST), 7. November 1982 (1982-11-07)

## Beschreibung

### Technisches Gebiet

Die Erfindung bezieht sich auf ein Biofeedback-Verfahren, bei dem einem einzelnen Nutzer zum Erlernen einer individuellen Entspannungstechnik die Herzrate oder der Hautwiderstand oder die Muskelspannung oder das Gleichstrom-Elektroencephalogramm als fortlaufend an seinem Körper gemessenes Biosignal optisch oder akustisch rückgemeldet wird.

### Stand der Technik

Aus DE 195 08 735 A1 ist eine Einrichtung zum Biofeedback-Training der Muskulatur eines Patienten bekannt. Hierbei werden in einem Speicher Patientendaten, Ist-, Soll- und Messwerte von Trainingsparametern, von Zeit- und Zählparametern und ein Auswertungsprogramm, insbesondere zur Inkontinenz-Therapie, gespeichert.

Die Sollwerte für die Trainingsparameter werden aus einer Istwertmessung eines Ruhewertes (Ruhetonus) bei entspannter Muskulatur des Patienten, einer maximalen Kontraktionskraft und einer die maximale Kontraktionskraft haltenden, maximalen Haltezeit in der Weise gebildet, dass mindestens eine Sollwertmarke für den Kontraktionssollwert aus der Multiplikation eines ersten Reduktionswertes kleiner eins mit dem um den Ruhewert reduzierten Wert der maximalen Kontraktionskraft und mindestens eine Sollwertmarke für den Haltezeit-Sollwert aus der Multiplikation eines zweiten Reduktionswertes kleiner eins mit der maximalen Haltezeit berechnet und die Sollwertmarken graphisch und/oder numerisch in einer Anzeigeeinheit angezeigt werden.

Aus EP 0 872 255 A ist eine Entspannungsleiteinrichtung bekannt, bei der am Körper eines Nutzers ein physiologisches Biosignal abgenommen wird, das dann gespeichert wird. Nach einiger Zeit wird der Person erneut ein der Art nach entsprechendes Biosignal abgenommen und mit dem vorher gespeicherten Signal verglichen. Die Veränderung über die Zeit wird analysiert, so dass festgestellt werden kann, ob sich der Zustand des Nutzers zu einem Zustand größerer Entspannung hin verbessert hat oder nicht. Dem Nutzer wird eine verbesserte Entspannung mitgeteilt, so dass er durch diese Kontrolle eine Entspannungstechnik einüben kann. Es handelt sich bei diesem Stand der Technik um die übliche Anwendung des Biofeedback-Verfahrens. Derartige Biofeedback-Verfahren werden in der Psychologie und Psychotherapie seit langem als Unterstützung zum Erlernen von individuellen Entspannungstechniken eingesetzt, wobei dem Nutzer ein an seinem Körper gemessenes Biosignal einer ausgewählten Körperfunktion, z.B. des Hautwiderstandes, rückgemeldet wird. Durch die aus EP 0 972 255 A bekannte, verbesserte Kontrolle wird vom Nutzer schneller eine Vorgehensweise zum Erreichen eines Entspannungszustandes erlernt und eingeübt als ohne technische Biofeedback-Unterstützung. Entspannungsübungen in Wettbewerbssituationen sind bei diesem bekannten Verfahren nicht möglich.

Bei einem aus US 5 720 619 A bekannten Verfahren handelt es sich um ein rechnergestütztes Multimedia-Biofeedbackspiel zum Beeinflussen der Spielerstimmung, d.h. der sogenannten Aura des Spielers, wobei mit einem softwaregesteuerten Prozessor eine veränderliche Spielerstimmung erzeugt wird, mit einem Bio-Joystick, der mit dem Prozessor kommuniziert, physiologische Variable des Spielers gemessen werden, auf einem Farb-Videomonitor, der ebenfalls mit dem Prozessor kommuniziert, in Echtzeit die veränderliche Stimmung des Spielers in Reaktion auf die physiologischen Variablen des Spielers dargestellt wird, eine Audiokomponente, die ebenfalls mit dem Prozessor kommuniziert, verbale Anweisungen an den Spieler zum Beeinflussen der physiologischen Variablen ausgibt, ein Modem, das ebenfalls mit dem Prozessor kommuniziert, die die physiologischen Variablen beinhaltenden Daten überträgt und Signalaussendemittel, die einen vom Spieler betrachteten Fernsehapparat einschließen, ein Signal bilden, das vom Modem zur Eingabe an den Prozessor empfangen wird. Es handelt sich hierbei um ein interaktives Spiel, bei dem sich die physiologischen Variablen ändern, wenn der Spieler durch die beobachtete Fernsehhandlung angeregt wird. Das Betrachten der Veränderungen seiner Stimmung, d.h. seiner Aura, ermöglicht es dem Spieler, die Steuerung unterbewusster Energien zu üben und sowohl die Rechnerausgabe als auch die Fernsehausgabe zu ändern. Es soll somit eine Stimmungsaufzeichnungseinrichtung mit einem interaktiven Rechnersystem integriert werden, so dass die Farbe der Stimmung, d.h. der Aura, den nachfolgenden Ablauf oder Ausgang des Computerspiels diktiert und der Spieler nicht nur die Farben der Stimmung steuern kann, wenn seine gemessenen und ebenfalls angezeigten physiologischen Variablen dem Spieler gezeigt werden, sondern den Ausgang des Programms auf solche Weise beeinflussen kann, dass Programme zur Erreichung eines gewünschten Zieles verändert werden. Ziel dieses bekannten Verfahren ist nicht das Erlernen einer individuellen Entspannungstechnik. Es lässt sich von einem Nutzer nicht die individuelle Entspannungsfähigkeit in Wettbewerbssituationen erlernen, verbessern und trainieren.

Bei einem aus US 5 213 338 A bekannten Verfahren handelt es sich um ein Spiel, das durch die Gehirnwellen von Spielern gesteuert wird und bei dem verschiedene Lichtmuster angezeigt oder bewegt werden, die von den Gehirnwellen gesteuert werden, die bei den Spielern erzeugt werden. Es handelt sich um ein elektronisches Vergnügungsspiel, das Mittel zum Detektieren von Gehirnwellenintensitäten der Spieler, Mittel zum Umwandeln der detektierten Gehirnwellenintensitäten in elektrische Impulse, deren Stärke den jeweiligen Gehirnwellenintensitäten proportional ist, eine drehbare Kreisplatte mit von der Drehzahl abhängiger wechselnder Sichtanzeige darauf und Mittel zum Drehen der Kreisplatte mit einer zur Stärke der jeweiligen Impulse proportionalen Drehzahl aufweist. Spielen beispielsweise zwei Personen gegeneinander, so wird die Kreisplatte proportional zur Stärke von denjenigen Impulsen aktiviert, die von den Gehirnwellen desjenigen Spielers stammen, der die stärkeren Gehirnwellen erzeugt. In einer Variante ist anstelle der Kreisplattendrehung auch die von den Gehirnwellen der Spieler gesteuerte Querbewegung eines Leuchtquadrats auf einem Anzeigetisch möglich, wobei zusätzlich den Spielern noch die zeitliche Veränderung der Gehirnwellensignale angezeigt wird. Bei dieser bekannten Einrichtung werden also die Gehirnwellen als gemessenes Biosignal erfasst und zur Steuerung eines Spiels verwendet. Mit diesem bekannten Verfahren kann ein einzelner Nutzer seine individuelle Entspannungsfähigkeit in Wettbewerbssituationen allerdings nicht ohne tatsächlich vorhandenen Gegner systematisch erlernen, verbessern und trainieren.

Nachfolgend wird eine kreative und neue Nutzung des seit langem bekannten Biofeedback-Verfahrens angegeben, das in der Psychologie und Psychotherapie seit langem als Unterstützung zum Erlernen von individuellen Entspannungstechniken genutzt wird. Hierbei wird dem einzelnen Nutzer ein an seinem Körper gemessenes Biosignal, sei dieses die Herzrate, der Hautwiderstand oder die Muskelspannung, optisch oder akustisch rückgemeldet. Erfahrungsgemäß wird hiermit schneller eine Vorgehensweise zum Erreichen der Entspannung erlernt als ohne eine solche technische Unterstützung.

Die Geräte, die im Zusammenhang mit der Anwendung des Biofeedback-Verfahrens gebraucht werden, sind häufig, z.B. für die Messung des Hautwiderstandes oder des Herzpulses, außergewöhnlich einfach und sind deswegen auch für die einzelnen Nutzer selbst erschwinglich.

### Darstellung der Erfindung

Der Erfindung liegt die Aufgabe zu Grunde, ein gattungsgemäßes Biofeedback-Verfahren dahingehend weiterzuentwickeln, dass die individuelle Entspannungsfähigkeit in Wettkampfsituationen ohne einen tatsächlich vorhandenen Gegner, also nur mit einem das Verfahren durchführenden Gerät, erlernt und geübt werden kann.

Gemäß der Erfindung, die sich auf ein Biofeedback-Verfahren der eingangs genannten Art bezieht, wird diese Aufgabe durch die in Anspruch 7 definierten Verfahrensschritte gelöst.

Der Kern des erfindungsgemäßen Biofeedback-Verfahrens ist darin zu sehen, dass ein Maß für die momentane Entspannungsfähigkeit rückgemeldet wird und dass die Höhe dieses Maßes durch eine gewollt herbeigeführte Konkurrenzsituation bezüglich der Entspannungsfähigkeit des Nutzers und des fiktiven programmierten Gegners beeinflusst werden kann. Hierdurch kann mit der Zeit eine bessere Entspannungsfähigkeit erlernt werden. Dabei spielt es keine wesentliche Rolle, welchen individuellen Weg der Nutzer wählt, um eine Entspannung bei sich selbst herbeizuführen.

Mit Hilfe des erfindungsgemäßen Biofeedback-Verfahrens können Personen, die unter Anspannung beispielsweise in einem Wettbewerb oder Spiel stehen, erfolgreicher trainiert werden und bei besonders stressanfälligen Personen kann eine weitgehende Stressvermeidung erreicht werden; d.h. diese Personen lernen, sich gerade in Wettkampfsituationen, in denen naturgemäß unwillkürlich körperliche Anspannung und Stress entstehen, willkürlich zu entspannen. Durch entsprechendes Einüben in allen Übungssituation kann diese Entspannung dann auf alle anderen Wettkampf-/Konkurrenzsituationen übertragen werden.

Der praktische Nutzen des Biofeedback-Verfahrens nach der Erfindung besteht zum einen darin, dass hiermit Personen, z.B. Hochleistungssportler, trainiert werden können, bei denen gerade die Verbindung von Wettkampfsituation und Entspannung für eine optimale sportliche Leistung besonders wichtig ist. Durch das regelmäßige Wettkampftraining mit dem Ziel des Erringens einer maximalen Entspannung könnte ein Lernerfolg im Sinne eines bedingten Reflexes erreicht werden, der dazu führt, dass durch dieses Training gerade unter Wettkampfbedingungen eine besonders intensive Entspannung erzielt wird.

Zum anderen besteht ein praktischer Nutzen des Biofeedback-Verfahrens nach der Erfindung darin, dass Personen, die besonders stressanfällig sind, d.h. sehr schnell in einen körperlichen Anspannungszustand geraten, durch gezieltes Erlernen einer Entspannungstechnik und damit rascheres Erreichen eines Entspannungszustandes hierdurch ihre Gesundheit fördern und Stressprävention betreiben können.

Eine nicht erfindungsgemäße Modifikation des Verfahrens besteht darin, dass Daten, die das eigene Leistungsniveau des einzelnen Nutzers als Inhalt haben, abgespeichert werden und dass diese abgespeicherten Leistungsniveaudaten als Leistungsniveaudaten des fiktiven programmierten Gegners herangezogen werden, gegen den der einzelne Nutzer dann konkurriert.

Insbesondere können auch diejenigen Daten, die das beste erzielte Leistungsniveau des einzelnen Nutzers zum Inhalt haben, abgespeichert werden, so dass dieser bei einer Konkurrenz gegen den abgespeicherten fiktiven programmierten Gegner sein momentanes Befinden (Tagesform) durch Vergleich mit seinem eigenen besten Befinden (Bestform) feststellen kann.

In zweckmäßiger Weise wird eine Kalibrierung der individuellen Eingangsdaten für das Biofeedback vorgenommen, was durch eine entsprechende Software erreicht werden kann.

Nicht sinnvoll ist eine solche Kalibrierung dann, wenn der betreffende einzelne Nutzer gegen sich selbst, d.h. gegen ein eingespeichertes eigenes Leistungsverhalten zu einem vorhergehenden Zeitpunkt, antritt.

In einer Einrichtung zur Durchführung des Biofeedback-Verfahrens nach der Erfindung ist zur Erstellung der Online-Kommunikationsverbindung zwischen dem einzelnen Nutzer und dem programmierten konkurrierenden Gegner ein Gerät vorgesehen, an das der einzelne Nutzer angeschlossen wird und in dem die Einrichtung zur Ausführung dieses Programms in zweckmäßiger Weise enthalten ist.

Die Online-Kommunikationsverbindung kann über eine elektrische Leitungsverbindung, über Infrarot-Schnittstellen, über Ultraschall, über Funk, im Rahmen eines Telefon-Festnetzes, über eine Funktelefonverbindung oder über ein Datenkommunikationsnetzwerk wie das Internet vorgenommen werden.

## Patentansprüche

1. Biofeedback-Verfahren, bei dem einem einzelnen Nutzer zum Erlernen einer individuellen Entspannungstechnik die Herzrate oder der Hautwiderstand oder die Muskelspannung oder das Gleichstrom-Elektroencephalogramm als fortlaufend an seinem Körper gemessenes Biosignal optisch oder akustisch rückgemeldet wird, wobei dem einzelnen Nutzer die zeitliche Veränderung seines Biosignals angezeigt wird, **dadurch gekennzeichnet, dass** das am Körper des einzelnen Nutzers gemessene momentane individuelle Biosignal mit einem momentanen individuellen Biosignal eines fiktiven programmierten Gegners über eine Online-Kommunikationsverbindung zusammengeführt und verglichen wird, und dass der fiktive programmierte Gegner so festprogrammiert wird, dass die zeitliche Veränderung seines Biosignals sich an der des Biosignals des einzelnen Nutzers bemisst und sich davon unterscheidet.

2. Biofeedback-Verfahren nach Anspruch 1, **dadurch gekennzeichnet, dass** eine Kalibrierung der individuellen, am Körper für das Biofeedback gemessenen Biosignale vorgenommen wird.

3. Biofeedback-Verfahren nach einem der vorhergehenden Ansprüche, **dadurch gekennzeichnet, dass** die Kommunikationsverbindung über eine elektrische Leitungsverbindung, über Infrarot-Schnittstellen, über Ultraschall, über Funk, im Rahmen eines Telefon-Festnetzes, über eine Funktelefonverbindung oder über ein Datenkommunikationsnetz wie das Internet vorgenommen wird.

## Claims

1. A biofeedback method whereby each individual user for learning an individual relaxation technique receives visual or audible feedback of the heart beat or skin resistance or muscular tension or the DC electroencephalogram as a bio signal measured continually on the user's body, the individual user receiving an indication of the change in time of his bio signal, **characterized in that** the individual bio signal measured at any one time on the body of the individual user is combined with and compared to the individual bio signal measured at any one time of a fictive programmed opponent via an online communications link and that the fictive programmed opponent is pre-programmed so that the change in time of his bio signal is referred to that of the bio signal of the individual user and differs therefrom.

2. The biofeedback method as set forth in claim 1, **characterized in that** the individual bio signals as measured on the body for the biofeedback are calibrated.

3. The biofeedback method as set forth in any of the preceding claims, **characterized in that** the communication link is made via electrical wiring, infrared interfaces, ultrasound, wireless, within the scope of a telephone network, via a radio telephone link or via a data communications network such as Internet

## Revendications

1. Procédé de biofeedback dans lequel le rythme cardiaque ou la résistance cutanée ou la tension musculaire ou bien l'électroencéphalogramme en courant continu est renvoyé de manière optique ou sonore à un utilisateur isolé en tant que biosignal mesuré en continu sur son corps pour l'apprentissage d'une technique de détente individuelle, la modification dans le temps de son biosignal étant signalée à l'utilisateur isolé, **caractérisé en ce que** le biosignal individuel instantané mesuré sur le corps de l'utilisateur isolé est rapproché et comparé avec un biosignal instantané individuel d'un adversaire fictif programmé par l'intermédiaire d'une liaison de communication en ligne, la modification dans le temps de son biosignal est mesurée sur celle du biosignal de l'utilisateur isolé et s'en distingue.

2. Procédé de biofeedback selon revendication 1, **caractérisé en ce qu'**on procède à un calibrage des biosignaux individuels mesurés sur le corps pour le biofeedback.

3. Procédé de biofeedback selon l'une des revendications précédentes, **caractérisé en ce que** la liaison de communication est assurée par un câble électrique, par des interfaces infrarouges, par ultrasons, par radio, dans le cadre d'un réseau téléphonique fixe, par une liaison téléphonique radio ou par un réseau de communication de données comme l'Internet.
